Europäisches Patentamt

European Patent Office

Office européen des brevets

(19)

(11) Veröffentlichungsnummer : **0 158 014**
**B1**

(12)  . **EUROPÄISCHE PATENTSCHRIFT**

(45) Veröffentlichungstag der Patentschrift :
19.08.87

(51) Int. Cl.⁴ : **A 61 F  2/36**

(21) Anmeldenummer : 85100943.1

(22) Anmeldetag : 30.01.85

(54) **Hüftgelenkprothese.**

(30) Priorität : 06.03.84 CH 1094/84

(43) Veröffentlichungstag der Anmeldung :
16.10.85 Patentblatt 85/42

(45) Bekanntmachung des Hinweises auf die Patenterteilung : 19.08.87 Patentblatt 87/34

(84) Benannte Vertragsstaaten :
**AT DE FR GB IT**

(56) Entgegenhaltungen :
EP-A- 0 058 745
EP-A- 0 093 230
CH-A-  600 866
DE-A- 2 839 093

(73) Patentinhaber : **GEBRÜDER SULZER AKTIENGESELL-
SCHAFT
Zürcherstrasse 9
CH-8401 Winterthur (CH)**

**Protek AG
Stadtbachstrasse 64
CH-3001 Bern (CH)**

(72) Erfinder : **Müller, Maurice E., Prof. Dr.-med.
Melchenbühlweg 9
CH-3006 Bern (CH)**

(74) Vertreter : **Dipl.-Ing. H. Marsch Dipl.-Ing. K. Sparing
Dipl.-Phys.Dr. W.H. Röhl Patentanwälte
Rethelstrasse 123
D-4000 Düsseldorf (DE)**

EP 0 158 014 B1

Jouve, 18, rue St-Denis, 75001 Paris, France

## Beschreibung

Die Erfindung betrifft eine Hüftgelenkprothese mit einem geraden, sich konisch erweiternden, blattartigen Schaft, dessen Blattseiten mindestens im proximalen Schaftteil mit einer, zu seiner Längsachse parallelen Rippenstruktur bedeckt sind, wobei im Uebergangsbereich zum Prothesenhals eine mit einer Einschieböffnung versehene Halskrause auf den Schaft aufschiebbar ist.

Ein derartiger Schaft ist aus der DE-A-3 216 538 bekannt ; die abnehmbare Halskrause, deren Abnehmbarkeit bei eingesetzter Prothese im Falle von Reoperationen das Entfernen des Schaftes aus dem Knochen erleichtern soll, wird dabei von medial nach lateral auf den vollständig in den Femurknochen eingesetzten Schaft der Prothese aufgeschoben.

Sollen Prothesenschäfte, die im Femur verankert sind, zusätzlich auf dem Rand der Operationsöffnung mit Hilfe eines Kragens oder einer Halskrause abgestützt werden, so ist es für eine wirksame Abstützung erforderlich, dass die Auflagefläche des Kragens und die Resektionsebene am Prothesenhals möglichst parallel zueinander verlaufen und darüberhinaus bezüglich eines Bezugspunktes die « richtige » Höhe haben, damit bei eingesetzter Prothese beispielsweise der Gelenkkopf relativ zum Trochanter auf dem richtigen Niveau gehalten wird. Diese beiden Forderungen haben bei der Verwendung bisheriger Prothesen zur Folge, dass der Resektionsschnitt sehr genau in der richtige Höhe und unter dem richtigen Winkel ausgeführt werden muss, was die Arbeit des Operateurs erheblich erschwert.

Aufgabe der Erfindung ist es daher, eine Prothese zu schaffen, bei der nachträglich Ungenauigkeiten in der Lage des Resektionsschnittes in einem gewissen Umfang korrigiert werden können. Diese Aufgabe wird nach der Erfindung dadurch gelöst, dass die Form und Abmessungen der Einschieböffnung derart an den Uebergangsbereich zum Prothesenhals angepasst sind, dass die Halskrause von distal her in Richtung der Längsachse des Schafts auf den Schaft aufschiebbar ist.

Auf diese Weise ist es möglich, einer Standard-Prothese, die gegebenenfalls auch ohne Halskrause verwendet werden kann bzw. einer bestimmten Schaftgrösse, einen ganzen Satz Halskrausen beizufügen, die unterschiedliche Dicken haben und/oder deren zur Auflage auf dem Knochen bestimmte Unterseite unterschiedliche Winkel mit der Prothesenhalsachse bilden. Aus diesem Satz Halskrausen kann der Operateur nach dem Resektionsschnitt die individuell am besten geeignete Halskrause auswählen, sie in Richtung der Längsachse auf den Schaft aufschieben und beim Einschlagen des Schaftes an diesem fixieren.

Für eine sichere Fixierung ist es vorteilhaft, wenn im medialen, rippenfreien Teil des Uebergangsbereichs ein konischer Klemmsitz zwischen Halskrause und Prothesenhals vorgesehen ist ; weiterhin können das Einschlagen des Schaftes, der sich durch Verklemmen im wesentlichen längs seines konischen Verlaufs abstützt, und das Fixieren der Halskrause erleichtert werden, wenn auf einer horizontalen Schulter am proximalen Schaftende zwei Einschlagbohrungen vorgesehen sind, von denen die eine mindestens nahezu im Bereich der Längsachse des Schaftes angeordnet ist, während sich die andere möglichst weit nach medial versetzt nahe dem Uebergang der Schulter in dem Prothesenhals befindet. Die nach medial gelegene Einschlagöffnung dient dabei in erster Linie dazu, die Halskrause auf den konischen Klemmsitz im medialen Uebergangsbereich zum Prothesenhals aufzukeilen.

Um die « Steilheit » des Resektionsschnittes und damit die Gefahr des « Abrutschens » der Halskrause bzw. der Prothese nach medial zu verringern, ist es weiterhin vorteilhaft, wenn mindestens die zur Auflage auf dem Knochen bestimmte Auflagefläche der aufgeschobenen Halskrause gegen die Prothesenhalsachse um einen von der Senkrechten abweichenden Winkel geneigt ist.

Schliesslich kann der Sitz der Prothese in bzw. auf dem Knochen zusätzlich verbessert werden, wenn die zur Auflage auf dem Knochen bestimmte Auflagefläche der Halskrause in ihrem nach medial gelegenen Teil mit scharfen keilartigen Zähnen belegt ist.

Im folgenden wird die Erfindung anhand eines Ausführungsbeispiels im Zusammenhang mit der Zeichnung näher erläutert.

Figur 1 ist eine ventral/dorsal gesehene Ansicht der neuen Prothese ;

Figur 2 gibt eine Ansicht von Fig. 1 von links wieder ;

Figur 3 ist, teilweise im Schnitt, eine Ansicht in Richtung des Pfeiles A von Fig. 1 ;

Figur 4 stellt eine Aufsicht auf Fig. 1 von oben dar, während

Figur 5 eine Aufsicht auf die Auflagefläche bzw. Unterseite einer Halskrause wiedergibt.

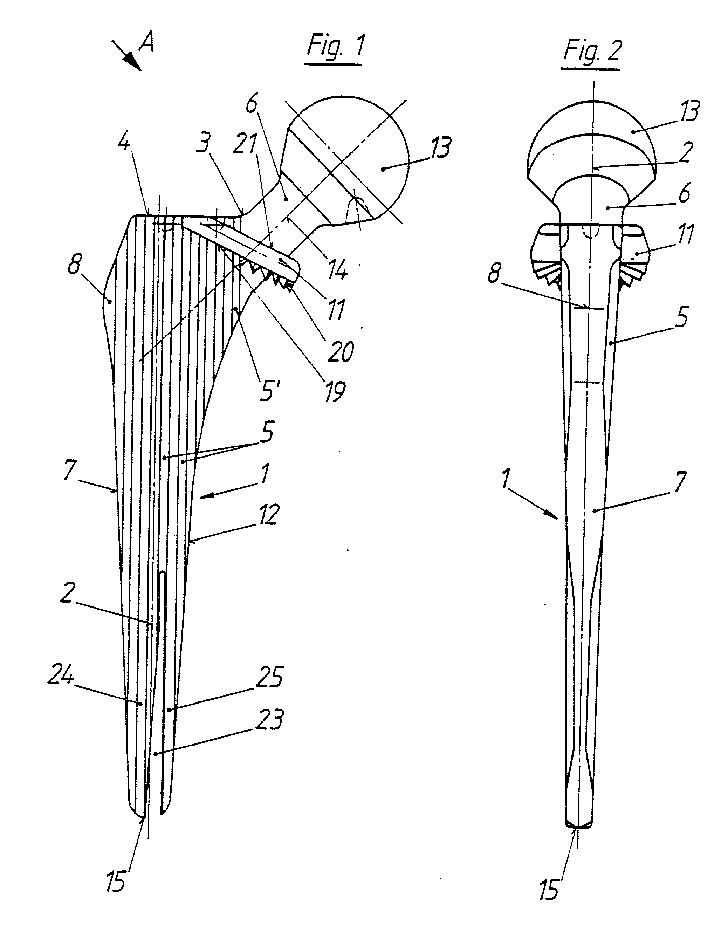

Die Blattseiten des Schaftes 1, die — wie in Fig. 2 gezeigt — über die ganze — längs der Mittelachse 2 gemessene Schafthöhe konisch erweitert verlaufen, sind mit einer aus sich parallel zur Mittelachse 2 erstreckenden Rillen bestehenden Oberflächenstruktur versehen, die das Anwachsen von Knochengewebe fördert ; diese Struktur reicht nach medial bis zu einem Punkt 3, in dem eine horizontale Schulter 4 in einen Prothesenhals 6 übergeht.

Auf den an diese Schulter 4 anschliessenden Prothesenhals 6 ist ein im wesentlichen kugelförmigen Gelenkkopf 13 aufgesetzt. Die Achse 14 des Prothesenhals 6 schneidet die Längsmittelachse des Schaftes 1 unter einem Winkel, der im wesentlichen dem Winkel zwischen dem Schenkelhals und der Femurachse eines natürlichen Hüftgelenks entspricht und im vorliegenden Fall etwa 45° betragt.

Die mediale Schmalseite 12 verläuft zunächst vom distalen Ende 15 aus konisch zur Mittelachse 2 und geht anschliessend in einen Bogen über, der in seinem Verlauf weitgehend dem Calcar-Bogen angepasst ist.

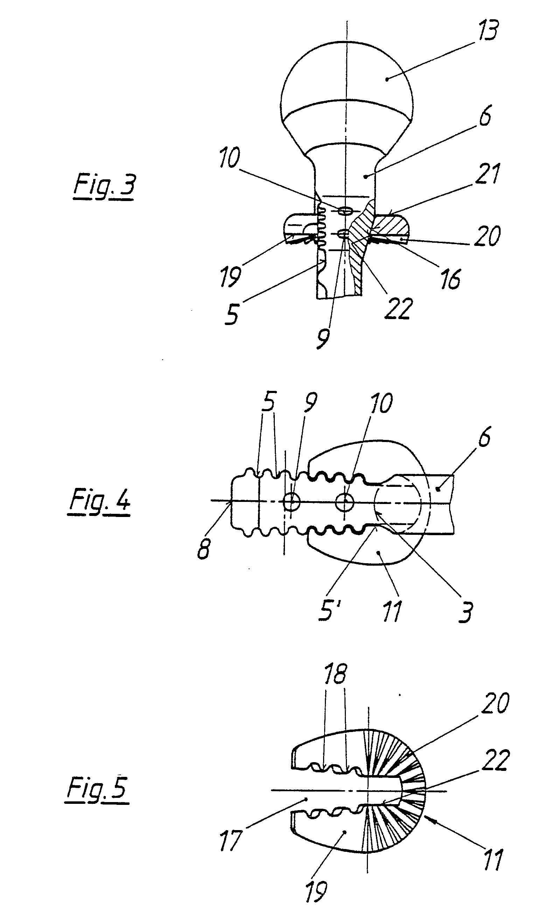

Aus der ebenfalls konisch verlaufenden lateralen Schmalseite 7 des Schaftes 1 springt — vom distalen Ende 15 aus etwas oberhalb 2/3 der Schafthöhe — in einem zunächst nach lateral gekrümmten Bogen ein Trochanterflügel 8 hervor, der in der horizontalen Schulter 4 endet. In ihr befinden sich zwei zueinander versetzte Einschlagöffnungen 9, 10 (Fig. 4) für den Einsatz eines Einschlaginstrumentes ; während die linke, nahe der Mittelachse 2 angeordnete Einschlagöffnung 9 zum Einschlagen des Schaftes 1 in den Knochen dient, hat die Oeffnung 10 die Aufgabe, mit Hilfe des Einschlaginstrumentes den Sitz einer Halskrause 11 auf dem Schaft 1 und ihren festen Sitz auf dem kortikalen Gewebe des Randes der Operationsöffnung zu sichern.  ·

Wie besonders aus Fig. 3 zu ersehen ist, hat die im wesentlichen hufeisenförmige Halskrause 11 eine Einschuböffnung 17, die in Form und Abmessungen an die Schaftkonturen im Uebergangsbereich zwischen der Oberflächenstruktur und dem Prothesenhals angepasst ist ; die parallel zur Mittelachse 2 verlaufenden Rillen 5 der Struktur enden, wie bereits erwähnt, nach medial in einer sich vom Punkt 3 parallel zur Mittelachse nach distal erstreckenden « letzten » Rille 5'. Der « glatte » Uebergangsbereich medial dieser Rille 5' bildet einen Konus 16 (Fig. 3), mit dem der Schaft in den etwas dickeren Prothesenhals 6 übergeht. Entsprechend diesem Uebergangsbereich ist die Einschuböffnung 17 in ihrem lateralen Teil mit zu den Rillen 5 komplementär ausgebildeten Rippen 18 versehen und bildet im medialen Teil einen Gegenkonus 22 zum Konus 16 (Fig. 3).

Die Auflagefläche oder Unterseite 19 (Fig. 5) der Halskrause 11, die im medialen Teil mit scharfen, keilartigen Zähnen 20 für ein Eindringen in den kortikalen « Rand » der Operationsöffnung versehen ist, kann mit der Oberseite 21 unterschiedliche Winkel bilden.

Bei der im Ausführungsbeispiel dargestellten « Normal-Form » der Halskrause 11 verlaufen Ober- und Unterseite 21 bzw. 19 parallel zueinander, wobei der Gegenkonus 22 relativ zu der Unterseite 19 so verläuft, dass diese lateral um etwa 20° gegenüber einer Ebene senkrecht zur Prothesenhalsachse 14 nach distal geneigt ist. Durch diese Massnahme wird der Winkel der Resektionsebene gegen eine Horizontale flacher, was die Gefahr des « Abrutschens » der Halskrause 11 vermindert. Von dieser Normal-Form mit parallelen Oberund Unterseiten 21 bzw. 19 aus kann die Unterseite 19 mit der Oberseite 21 negative oder positive Winkel einschliessen, wobei bei positiven Winkeln diese beiden Flächen der Halskrause 11 nach lateral auseinander und bei negativen Winkeln aufeinander zulaufen.

Parallel zur Mittelachse 2 ist am distalen Ende 15 des Schaftes 1 zur Anpassung des distalen Schaftteils an unterschiedlich weite operativ geschaffene Hohlräume im Femurknochen ein Längsschlitz 23 vorgesehen, der eine elastische und/oder plastische Verformung des distalen Schaftteils erlaubt ; dieser Längsschlitz 23 ist aus der Mittelachse 2 heraus nach medial so versetzt, dass sein laterale Schenkel 24. trotz einer Erweiterung nach lateral/distal — praktisch starr und unverformbar ist und die ganze Verformung im medialen Schenkel 25 erfolgt.

## Patentansprüche

1. Hüftgelenkprothese mit einem geraden, sich konisch erweiternden, blattartigen Schaft (1), dessen Blattseiten mindestens im proximalen Schaftteil mit einer, zu seiner Längsachse (2) parallelen Rippenstruktur (5) bedeckt sind, wobei im Uebergangsbereich zum Prothesenhals (6) eine mit einer Einschieböffnung (17) versehene Halskrause auf den Schaft aufschiebbar ist, dadurch gekennzeichnet, dass die Form und Abmessungen der Einschieböffnung derart an den Uebergangsbereich zum Prothesenhals (6) angepasst sind, dass die Halskrause (11) von distal her in Richtung der Längsachse (2) des Schafts auf den Schaft (1) aufschiebbar ist.

2. Hüftgelenkprothese nach Anspruch 1, dadurch gekennzeichnet, dass im medialen rippenfreien Teil des Ueberganskbereiches ein konischer Klemmsitz (16, 22) zwischen Halskrause (11) und Prothesenhals (6) vorgesehen ist.

3. Hüftgelenkprothese nach Anspruch 1 oder 2, dadurch gekennzeichnet, dass mindestens die zur Auflage auf dem Knochen bestimmte Auflagefläche (19) der aufgeschobenen Halskrause (11) gegen die Prothesenhalsachse (14) um einen von der Senkrechten abweichenden Winkel geneigt ist.

4. Hüftgelenkprothese nach einem der Ansprüche 1 bis 3, dadurch gekennzeichnet, dass die zur Auflage auf dem Knochen bestimmte Auflagefläche (19) der Halskrause (11) in ihrem nach medial gelegenen Teil mit scharfen keilartigen Zähnen (20) belegt ist.

5. Hüftgelenkprothese nach einem der Ansprüche 1 bis 4, dadurch gekennzeichnet, dass auf einer horizontalen Schulter (4) am proximalen Schaftende zwei Einschlagbohrungen (9, 10) vorgesehen sind, von denen die eine mindestens nahezu im Bereich der Längsachse (2) des Schaftes (1) angeordnet ist, während sich die andere möglichst weit nach medial versetzt nahe dem Uebergang der Schulter (4) in dem Prothesenhals (6) befindet.

## Claims

1. A hip joint replacement having a straight conically widening blade-like stem (1), the blade sides being covered at least in the proximal stem part by a rib structure (5) parallel to the stem longitudinal axis (2), a ruff formed with an insertion aperture (17) being adapted to be pushed on to the stem in the region of the transition to the neck of the replacement joint, characterised in that the shape and dimensions of the insertion aperture are so adapted to the region of transition to the neck (6) of the joint replacement that the ruff (11) can be pushed on to the stem (1) from the distal side in the direction of the stem longitudinal axis (2).

2. A joint according to claim 1, characterised in that there is a conical clamping fit (16, 22) between the ruff (11) and neck (6) in the medial unribbed part of the transition zone.

3. A joint according to claim 1 or 2, characterised in that at least that surface (19) of the pushedon ruff (11) which is intended to bear on the bone is inclined relatively to the joint neck axis (14) by an angle other than a right angle.

4. A joint according to any of claims 1-3, characterised in that that bearing surface (19) of the ruff (11) which is intended to bear on the bone has in its medial part sharp wedge-like teeth (20).

5. A joint according to any of claims 1-4, characterised in that a horizontal shoulder (4) at the proximal stem end is formed with two knock-in bores (9, 10), one of which is disposed at least substantially near the stem longitudinal axis (2) while the other is disposed, offset as far as possible in the medial direction, near the transition between the shoulder (4) and the neck (6).

**Revendications**

1. Prothèse coxofémorale munie d'une tige rectiligne (1) en forme de spatule à élargissement tronconique, dont les côtés de la spatule sont recouverts, au moins dans la partie proximale de la tige, par une structure striée (5) parallèle à son axe longitudinal (2), une collerette percée d'un orifice d'enfilement (17) pouvant être enfilée sur la tige, dans la zone de transition avec le col (6) de la prothèse, caractérisée par le fait que la forme et les dimensions de l'orifice d'enfilement sont adaptées à la zone de transition avec le col (6) de la prothèse, de telle sorte que la collerette (11) puisse être enfilée sur la tige (1) à partir du côté distal, en direction de l'axe longitudinal (2) de la tige.

2. Prothèse coxofémorale selon la revendication 1, caractérisée par le fait qu'une assise tronconique par coincement (16, 22) est prévue, entre la collerette (11) et le col (6) de la prothèse, dans la partie médiale de la zone de transition dépourvue de striures.

3. Prothèse coxofémorale selon la revendication 1 ou 2, caractérisée par le fait qu'au moins la surface d'appui (19) de la collerette enfilée (11), destinée à reposer sur l'os, est inclinée par rapport à l'axe (14) du col de la prothèse, d'un angle s'écartant de la verticale.

4. Prothèse coxofémorale selon l'une des revendications 1 à 3, caractérisée par le fait que la surface d'appui (19) de la collerette (11), destinée à reposer sur l'os, est garnie de dents pointues (20) du type ardillons dans sa partie située du côté médial.

5. Prothèse coxofémorale selon l'une des revendications 1 à 4, caractérisée par le fait que deux trous (9, 10) d'enfoncement par percussion sont prévus sur un épaulement horizontal (4) à l'extrémité proximale de la tige, trous dont l'un est disposé au moins approximativement au voisinage de l'axe longitudinal (2) de la tige (1), tandis que l'autre, décalé le plus loin possible vers le côté médial, se trouve à proximité de la transition entre l'épaulement (4) et le col (6) de la prothèse.

Fig. 1

Fig. 2

_Fig. 3_

_Fig. 4_

_Fig. 5_

2